# EUROPEAN PATENT SPECIFICATION

(11) **EP 3 166 582 B1**
(45) Date of publication and mention of the grant of the patent: **29.01.2020**
(21) Application number: 15731595.3
(22) Date of filing: 26.06.2015
(51) Int. Cl.: A61K 8/44, A61K 8/49, A61K 8/362, A61Q 5/00

(54) **USE OF SPICULISPORIC ACID AND/OR A SALT THEREOF AS ANTIDANDRUFF AGENT, AND COSMETIC TREATMENT PROCESS EMPLOYING SAME**
VERWENDUNG VON SPICULISPORSÄURE UND/ODER EINEM SALZ DARAUS ALS ANTISCHUPPENMITTEL UND KOSMETISCHES BEHANDLUNGSVERFAHREN DAMIT
UTILISATION D'ACIDE SPICULISPORIQUE ET/OU D'UN SEL DE CELUI-CI EN TANT QU'AGENT ANTIPELLICULAIRE ET PROCÉDÉ DE TRAITEMENT COSMÉTIQUE LE/S UTILISANT

(30) Priority: 10.07.2014 FR 1456666
(43) Date of publication of application: 17.05.2017
(73) Proprietor: L'Oréal, 75008 Paris (FR)
(72) Inventor: SIRICHANDRA, Caroline, 94340 Joinville le Pont (FR); LESCH, Sandie, 94152 Chevilly Larue (FR)
(74) Representative: L'Oreal
(86) International application number: PCT/EP2015/064494
(87) International publication number: WO 2016/005209

(56) References cited:
- EP-A1- 2 055 314
- WO-A1-2015/067784
- RAHMAN P K S M ET AL: "Production, Characterisation and Applications of Biosurfactants-Review", BIOTECHNOLOGY, ASIAN NETWORK FOR SCIENTIFIC INFORMATION, PK, vol. 7, no. 2, 31 December 2008 (2008-12-31), pages 360-370, XP002614719, ISSN: 1682-296X, DOI: 10.3923/BIOTECH.2008.360.370
- ISHIGAMI Y ET AL: "Surface active properties of biosoap from spiculisporic acid", JOURNAL OF COLLOID AND INTERFACE SCIENCE, ACADEMIC PRESS, NEW YORK, NY, US, vol. 94, no. 1, 1 July 1983 (1983-07-01), pages 131-139, XP024187869, ISSN: 0021-9797, DOI: 10.1016/0021-9797(83)90242-4 [retrieved on 1983-07-01]
- DATABASE WPI Week 200763 Thomson Scientific, London, GB; AN 2007-671942 XP002739286, & KR 2006 0111183 A (DONGWOO FINE CHEM CO LTD) 26 October 2006 (2006-10-26)

## Description

The present invention relates to the cosmetic use, especially for hair, of spiculisporic acid and/or a salt thereof, as antidandruff agent, in particular in the cosmetic treatment of dandruff conditions associated with the excessive proliferation of yeasts of the Malassezia genus on the scalp.

Dandruff problems affect up to 50% of the worldwide population. They affect both men and women and are perceived as having a very negative psychosocial impact. The appearance of dandruff is disagreeable both aesthetically and because of the discomfort it causes (especially tingling or itching), and as such many people confronted with this problem wish to eliminate it efficiently and permanently.
Dandruff corresponds to excessive and visible desquamation of the scalp resulting from excessively rapid multiplication of the epidermal cells and their abnormal maturation. This phenomenon may be caused especially by excessively aggressive hair treatments, extreme climatic conditions, stress, diet, fatigue and pollution. Dandruff conditions usually result from a disorder of the scalp microflora and more particularly from excessive colonization by a fungus which belongs to the family of yeasts of the Malassezia genus and which is naturally present on the scalp.
Many antidandruff treatments have been developed with the principal objective of eradicating Malassezia yeasts from the scalp. Thus, the activity of the antidandruff active agents currently used, such as zinc pyrithione, piroctone olamine or selenium disulfide, is based mainly on their fungicidal property. However, these antidandruff agents are not completely satisfactory in terms of effectiveness (immediate effectiveness and/or duration of the effect) and/or in terms of their impact on the environment.
Thus, finding solutions to formulate environmentally friendly cosmetic products which are just as effective has become a major challenge in order to respond to the needs of consumers.

An aim of the present invention is to provide an antidandruff agent that is non-irritant to the skin and the scalp, and that is as effective as the known antidandruff agents, while having a more favourable environmental impact, especially fulfilling criteria of naturalness and/or renewability.
Another aim of the invention is to propose an active agent that can re-establish, and maintain at a normal level, the ecoflora of the scalp and especially prevent excessive colonization of the scalp by Malassezia sp.

The Applicant has discovered surprisingly that the cosmetic use of spiculisporic acid and/or a salt thereof enabled effective treatment of dandruff conditions, especially those associated with the proliferation of yeasts of the Malassezia genus. It was observed that, by using the compound according to the invention, the excessive number of yeasts of the Malassezia genus could be reduced and consequently the number of dandruff could be reduced.
Spiculisporic acid is a natural and environmentally friendly biosurfactant from a renewable source and is readily biodegradable.

A subject-matter of the present invention is therefore the cosmetic use of spiculisporic acid and/or a salt thereof as antidandruff agent.
Another subject-matter of the present invention is a cosmetic treatment process intended for eliminating dandruff and/or for reducing the amount thereof, comprising the application, to the hair and/or the scalp, of spiculisporic acid and/or a salt thereof, or of a cosmetic composition comprising it/them.

### Spiculisporic acid

Spiculisporic acid, also known by the name 4,5-dicarboxy-4-pentadecanolide, has the following formula:

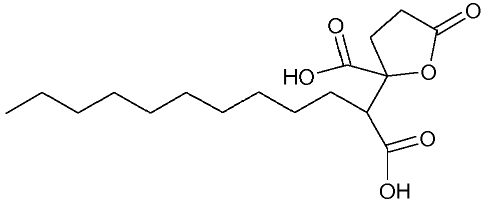

The spiculisporic acid which is usable in the context of the invention may be in salified or unsalified form.
When it is in the form of a salt, it may be an organic or inorganic salt. In this case, the spiculisporic acid may be used in a mixture with an organic or inorganic base. This base is a neutralizing base, that is to say it enables the spiculisporic acid to be neutralized so as to form a salt of said acid. In particular, the bases may be selected from:
- aqueous ammonia,
- alkanolamines, such as mono-, di- and triethanolamines, isopropanolamine and 2-amino-2-methyl-1-propanol,
- oxyethylenated and/or oxypropylenated ethylenediamines,
- inorganic or organic hydroxides.
The bases are preferably selected from hydroxides of an alkali metal, hydroxides of an alkaline-earth metal, for instance sodium hydroxide or potassium hydroxide, hydroxides of a transition metal, such as hydroxides of metals from Groups III, IV, V and VI of the Periodic Table of the Elements, hydroxides of lanthanides or actinides, quaternary ammonium hydroxides and guanidinium hydroxide. The hydroxide may be formed *in situ*, such as for instance guanidine hydroxide, formed by reacting calcium hydroxide and guanidine carbonate.
- alkali metal silicates, such as sodium metasilicates,
- amino acids, preferably basic amino acids, such as arginine, lysine, ornithine, citrulline and histidine,
- carbonates and bicarbonates, particularly of a primary amine, secondary amine or tertiary amine, of an alkali metal or alkaline-earth metal, or of ammonium, and
- the compounds of following formula: 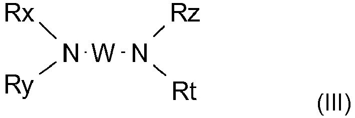 in which W is a C₁-C₆ alkylene residue optionally substituted with a hydroxyl group or a C₁-C₆ alkyl group; Rx, Ry, Rz and Rt, which may be identical or different, represent a hydrogen atom or a C₁-C₆ alkyl, C₁-C₆ hydroxyalkyl or C₁-C₆ aminoalkyl group. Mention may especially be made of 1,3-diaminopropane, 1,3-diamino-2-propanol, spermine and spermidine.

Most particularly, the base may be selected from inorganic hydroxides, such as sodium hydroxide, potassium hydroxide, lithium hydroxide, caesium hydroxide, calcium hydroxide, magnesium hydroxide, zinc hydroxide; basic amino acids, such as lysine, arginine, histidine, citrulline, ornithine; alkanolamines, such as monoethanolamine, diethanolamine, 2-(dimethylamino)ethanol, triethanolamine, triisopropanolamine, diisopropanolamine, monoisopropanolamine; aqueous ammonia; guanidine carbonate; and mixtures thereof.
Preferably, the base is selected from arginine, triethanolamine, potassium hydroxide, sodium hydroxide and mixtures thereof.

Thus, it is most particularly preferred to use the sodium, potassium, triethanolamine and/or arginine salts of spiculisporic acid.

Preferably, the weight ratio spiculisporic acid/base is between 1/0.5 and 1/1.1, preferably from 1/0.6 to 1/0.8, and better still from 1/0.6 to 1/0.7.

### Cosmetic composition

The spiculisporic acid may be carried in a cosmetic composition so as to be applied to the hair and/or the scalp.
Preferably, said cosmetic composition comprises a cosmetically acceptable medium, that is to say a medium compatible with topical application to keratin materials, especially the scalp and the hair.

Preferably, the pH of said cosmetic composition is between 4 and 7.5, notably between 4.5 and 7, and in particular between 4.7 and 6.5.

Preferably, the spiculisporic acid and/or the salts thereof is present in the cosmetic composition in an amount of between 0.5 and 20% by weight, in particular between 1 and 15% by weight, more particularly between 1.5 and 10% by weight, relative to the total weight of the composition.

Preferably, said cosmetic composition is a composition for the hair, which may be a rinse-out or a leave-in composition. The composition is preferably in the form of a shampoo, a cream, a mousse (aerosol or non-aerosol), a paste, a gel, an emulsion, a lotion or even a stick. Preferably, the composition for the hair is a shampoo, a gel or a lotion.

The cosmetic composition preferably comprises water and/or one or more organic solvents that may be selected from linear or branched C₁-C₆ monoalcohols, such as ethanol, isopropanol, tert-butanol or n-butanol; polyols, such as glycerol, propylene glycol, hexylene glycol (or 2-methyl-2,4-pentanediol) and polyethylene glycols; polyol ethers, such as dipropylene glycol monomethyl ether; and mixtures thereof.
Preferably, the cosmetic composition comprises water in an amount ranging from 30 to 98% by weight, especially from 40 to 95% by weight, better from 50 to 90% by weight, relative to the total weight of the composition.
Preferably, the cosmetic composition comprises the organic solvent(s) in an amount ranging from 0.05 to 60%, preferably from 0.5 to 50% and better still from 1 to 40% by weight, relative to the total weight of the cosmetic composition.

Said cosmetic composition according to the invention may also comprise at least one customary cosmetic ingredient, especially selected from plant, mineral, animal or synthetic oils; liquid fatty alcohols; liquid fatty esters; solid fatty substances and notably waxes, solid fatty esters, solid alcohols; anionic, cationic, amphoteric and nonionic surfactants; anionic, nonionic, amphoteric and cationic polymers; sunscreens; moisturizers; antidandruff agents other than spiculisporic acid; antioxidants; agents for combating hair loss; pearlizing and opacifying agents; plasticizers or coalescence agents; hydroxy acids; fillers; silicones and in particular polydimethylsiloxanes (PDMSs); perfumes; basifying agents or acidifying agents; aldehydes, DHA; polymeric or non-polymeric thickeners, and notably associative polymers; preservatives; sequestrants (EDTA and salts thereof); colorants. The composition can, of course, comprise several cosmetic ingredients appearing in the above list. A person skilled in the art will take care to select the ingredients making up the composition, and also the amounts thereof, such that the advantageous properties of the composition according to the invention are not, or are not substantially, adversely affected by the envisaged addition.

The application of said acid or a salt thereof or the composition comprising same to keratin materials, such as the hair and/or the scalp, can be followed, or not, by a rinsing step, for example with water.

The invention is illustrated in more detail in the following examples.

### Example 1: demonstration of activity on Malassezia furfur

### Protocol:

- The model strain used is: *Malassezia furfur* ATCC 12078 (yeast).
- The strain is placed in contact with the formula to be tested in a suitable liquid culture medium in the following ratios:
   10% of the microbial inoculum at 10⁷ microorganisms/ml
   10% of the formula to be tested
   80% of liquid culture medium (sabouraud broth + olive oil).
- In parallel, a growth control, in which the formula to be tested is replaced with diluent (tryptone salt), is prepared under the same conditions.
- The samples are placed in a rotary incubator at 35°C and stirred throughout the duration of the test.

After 2, 6 and 24 hours of contact, the number of revivable microorganisms remaining in the mixture is evaluated.

The results are expressed as a logarithm of the number of microorganisms per millilitre of mixture.

### Results:

### 1/

### Formula A comprising (% by weight):

- 10% spiculisporic acid
- 5.84% L-arginine (R_{base}/R_{acid} ratio = 1.1)
- water q.s. 100%

Change in the number of revivable microorganisms per ml of sample (in log):

| | t0 | t2h | t6h | t24h |
|---|---|---|---|---|
| Formula A | 6.0 | 5.3 | 4.7 | 2.4 |
| Control | 6.0 | 5.6 | 5.8 | 5.8 |

It is observed that, relative to the growth control, formula A according to the invention has good antimicrobial activity on M. furfur (reduction by 3.4 log units at 24 hours).

### 2/

### Formula B comprising (% by weight):

- 5% spiculisporic acid
- 2.92% L-arginine (R_{base}/R_{acid} ratio = 1.1)
- water q.s. 100%

Change in the number of revivable microorganisms per ml of sample (in log):

| | t0 | t2h | t6h | t24h |
|---|---|---|---|---|
| Formula B | 6.0 | 5.3 | 4.8 | 2.1 |
| Control | 6.0 | 5.6 | 5.8 | 5.8 |

It is observed that, relative to the growth control, formula B according to the invention has good antimicrobial activity on M. furfur (reduction by 3.7 log units at 24 hours).

### Example 2

A hair lotion is prepared, comprising (% by weight):

| | |
|---|---|
| - spiculisporic acid | 5% |
| - L-arginine | 3.1% |
| - water | qsp 100% |

This lotion can be applied to the hair and make it possible to combat dandruff.

### Example 3

An antidandruff shampoo is prepared, comprising (% by weight):

| | |
|---|---|
| - sodium lauryl sulfate | 10% |
| - cocoylbetaine | 5% |
| - cocamide MEA | 1% |
| - spiculisporic acid | 2% |
| - L-arginine | 1.25% |
| - preservative | q.s. |
| - water | qsp 100% |

## Claims

1. Cosmetic use, especially for hair, of spiculisporic acid and/or a salt thereof as antidandruff agent.

2. Use according to Claim 1, in which spiculisporic acid is used in a mixture with an organic or inorganic base, preferably selected from:
- aqueous ammonia,
- alkanolamines, such as mono-, di- and triethanolamines, isopropanolamine and 2-amino-2-methyl-1-propanol,
- oxyethylenated and/or oxypropylenated ethylenediamines,
- inorganic or organic hydroxides,
- alkali metal silicates, such as sodium metasilicates,
- amino acids, preferably basic amino acids, such as arginine, lysine, ornithine, citrulline and histidine,
- carbonates and bicarbonates, particularly of a primary amine, secondary amine or tertiary amine, of an alkali metal or alkaline-earth metal, or of ammonium, and
- the compounds of following formula: 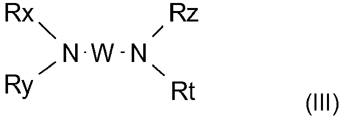 in which W is a C₁-C₆ alkylene residue optionally substituted with a hydroxyl group or a C₁-C₆ alkyl group; Rx, Ry, Rz and Rt, which may be identical or different, represent a hydrogen atom or a C₁-C₆ alkyl, C₁-C₆ hydroxyalkyl or C₁-C₆ aminoalkyl group; such as 1,3-diaminopropane, 1,3-diamino-2-propanol, spermine or spermidine;
preferably, the base is selected from inorganic hydroxides, such as sodium hydroxide, potassium hydroxide, lithium hydroxide, caesium hydroxide, calcium hydroxide, magnesium hydroxide, zinc hydroxide; basic amino acids, such as lysine, arginine, histidine, citrulline, ornithine; alkanolamines, such as monoethanolamine, diethanolamine, 2-(dimethylamino)ethanol, triethanolamine, triisopropanolamine, diisopropanolamine, monoisopropanolamine; aqueous ammonia; guanidine carbonate; and mixtures thereof;
and even better still the base is selected from arginine, triethanolamine, potassium hydroxide, sodium hydroxide and mixtures thereof.

3. Use according to either of the preceding claims, in which the spiculisporic acid and/or the salts thereof is carried in a cosmetic composition in which it is present in an amount of between 0.5 and 20% by weight, in particular between 1 and 15% by weight, more particularly between 1.5 and 10% by weight, relative to the total weight of the composition.

4. Use according to Claim 3, in which said cosmetic composition comprises water and/or one or more organic solvents that may be selected from linear or branched C₁-C₆ monoalcohols, such as ethanol, isopropanol, tert-butanol or n-butanol; polyols, such as glycerol, propylene glycol, hexylene glycol (or 2-methyl-2,4-pentanediol) and polyethylene glycols; polyol ethers, such as dipropylene glycol monomethyl ether; and mixtures thereof.

5. Use according to either of Claims 3 and 4, in which said cosmetic composition comprises water in an amount ranging from 30 to 98% by weight, especially from 40 to 95% by weight, better from 50 to 90% by weight, relative to the total weight of the composition; and/or the cosmetic composition comprises the organic solvent(s) in an amount ranging from 0.05 to 60%, preferably from 0.5 to 50% and better still from 1 to 40% by weight, relative to the total weight of the cosmetic composition.

6. Use according to one of Claims 3 to 5, in which said cosmetic composition comprises at least one customary cosmetic ingredient, especially selected from plant, mineral, animal or synthetic oils; liquid fatty alcohols; liquid fatty esters; solid fatty substances and notably waxes, solid fatty esters, solid alcohols; anionic, cationic, amphoteric and nonionic surfactants; anionic, nonionic, amphoteric and cationic polymers; sunscreens; moisturizers; antidandruff agents other than spiculisporic acid; antioxidants; agents for combating hair loss; pearlizing and opacifying agents; plasticizers or coalescence agents; hydroxy acids; fillers; silicones and in particular polydimethylsiloxanes (PDMSs); perfumes; basifying agents or acidifying agents; aldehydes, DHA; polymeric or non-polymeric thickeners, and notably associative polymers; preservatives; sequestrants (EDTA and salts thereof); colorants.

7. Use according to one of Claims 3 to 6, in which the pH of said cosmetic composition is between 4 and 7.5, notably between 4.5 and 7, and in particular between 4.7 and 6.5.

8. Use according to one of Claims 3 to 7, in which said cosmetic composition is a composition for the hair, which may be in the form of a shampoo, a cream, an aerosol or non-aerosol mousse, a paste, a gel, an emulsion, a lotion or a stick.

9. Cosmetic treatment process for eliminating dandruff and/or for reducing the amount thereof, comprising the application, to the hair and the scalp, of spiculisporic acid and/or a salt thereof as antidandruff agent.

10. Process according to Claim 9, in which spiculisporic acid is used in a mixture with an organic or inorganic base, preferably selected from:
- aqueous ammonia,
- alkanolamines, such as mono-, di- and triethanolamines, isopropanolamine and 2-amino-2-methyl-1-propanol,
- oxyethylenated and/or oxypropylenated ethylenediamines,
- inorganic or organic hydroxides,
- alkali metal silicates, such as sodium metasilicates,
- amino acids, preferably basic amino acids, such as arginine, lysine, ornithine, citrulline and histidine,
- carbonates and bicarbonates, particularly of a primary amine, secondary amine or tertiary amine, of an alkali metal or alkaline-earth metal, or of ammonium, and
- the compounds of following formula: 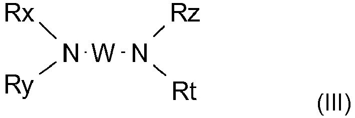 in which W is a C₁-C₆ alkylene residue optionally substituted with a hydroxyl group or a C₁-C₆ alkyl group; Rx, Ry, Rz and Rt, which may be identical or different, represent a hydrogen atom or a C₁-C₆ alkyl, C₁-C₆ hydroxyalkyl or C₁-C₆ aminoalkyl group; such as 1,3-diaminopropane, 1,3-diamino-2-propanol, spermine or spermidine;
preferably, the base is selected from inorganic hydroxides, such as sodium hydroxide, potassium hydroxide, lithium hydroxide, caesium hydroxide, calcium hydroxide, magnesium hydroxide, zinc hydroxide; basic amino acids, such as lysine, arginine, histidine, citrulline, ornithine; alkanolamines, such as monoethanolamine, diethanolamine, 2-(dimethylamino)ethanol, triethanolamine, triisopropanolamine, diisopropanolamine, monoisopropanolamine; aqueous ammonia; guanidine carbonate; and mixtures thereof;
and better still the base is selected from arginine, triethanolamine, potassium hydroxide, sodium hydroxide and mixtures thereof.

11. Process according to either of Claims 9 and 10, in which the spiculisporic acid and/or the salts thereof is present in the cosmetic composition in an amount of between 0.5 and 20% by weight, in particular between 1 and 15% by weight, more particularly between 1.5 and 10% by weight, relative to the total weight of the composition.

12. Process according to one of Claims 9 to 11, in which said cosmetic composition comprises water and/or one or more organic solvents that may be selected from linear or branched C₁-C₆ monoalcohols, such as ethanol, isopropanol, tert-butanol or n-butanol; polyols, such as glycerol, propylene glycol, hexylene glycol (or 2-methyl-2,4-pentanediol) and polyethylene glycols; polyol ethers, such as dipropylene glycol monomethyl ether; and mixtures thereof;
notably said cosmetic composition comprises water in an amount ranging from 30 to 98% by weight, especially from 40 to 95% by weight, better still from 50 to 90% by weight, relative to the total weight of the composition; and/or the cosmetic composition comprises the organic solvent(s) in an amount ranging from 0.05 to 60%, preferably from 0.5 to 50%, and better still from 1 to 40% by weight, relative to the total weight of the cosmetic composition.

13. Process according to one of Claims 9 to 12, in which said cosmetic composition comprises at least one customary cosmetic ingredient, especially selected from plant, mineral, animal or synthetic oils; liquid fatty alcohols; liquid fatty esters; solid fatty substances and notably waxes, solid fatty esters, solid alcohols; anionic, cationic, amphoteric and nonionic surfactants; anionic, nonionic, amphoteric and cationic polymers; sunscreens; moisturizers; antidandruff agents other than spiculisporic acid; antioxidants; agents for combating hair loss; pearlizing and opacifying agents; plasticizers or coalescence agents; hydroxy acids; fillers; silicones and in particular polydimethylsiloxanes (PDMSs); perfumes; basifying agents or acidifying agents; aldehydes, DHA; polymeric or non-polymeric thickeners, and notably associative polymers; preservatives; sequestrants (EDTA and salts thereof); colorants.

14. Process according to one of Claims 9 to 13, in which the pH of said cosmetic composition is between 4 and 7.5, notably between 4.5 and 7, and in particular between 4.7 and 6.5.

15. Process according to one of Claims 9 to 14, in which said cosmetic composition is a composition for the hair, which may be in the form of a shampoo, a cream, an aerosol or non-aerosol mousse, a paste, a gel, an emulsion, a lotion or a stick.

## Patentansprüche

1. Kosmetische Verwendung von Spiculosporsäure und/oder einem Salz davon als Antischuppenmittel, insbesondere für Haar.

2. Verwendung nach Anspruch 1, wobei Spiculosporsäure in einer Mischung mit einer organischen oder anorganischen Base verwendet wird, welche vorzugsweise aus
- wässrigem Ammoniak,
- Alkanolaminen, wie Mono-, D- und Triethanolaminen, Isopropylamin und 2-Amino-2-methyl-1-propanol,
- oxyethylenierten und/oder oxypropylenierten Ethylendiaminen,
- anorganischen oder organischen Hydroxiden,
- Alkalimetallsilikaten, wie Natriummetasilikaten,
- Aminosäuren, vorzugsweise basischen Aminosäuren, wie Arginin, Lysin, Ornithin, Citrullin und Histidin,
- Carbonaten und Hydrogencarbonaten, insbesondere von einem primären Amin, sekundären Amin oder tertiären Amin, von einem Alkalimetall oder Erdalkalimetall oder von Ammonium, und
- den Verbindungen der folgenden Formel: 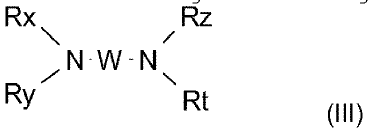 wobei W für einen C₁-C₆-Alkylenrest, der gegebenenfalls durch eine Hydroxylgruppe oder eine C₁-C₆-Alkylgruppe substituiert ist, steht; Rx, Ry, Rz und Rt, die gleich oder verschieden sein können, für ein Wasserstoffatom oder eine C₁-C₆-Alkyl-, C₁-C₆-Hydroxyalkyl- oder C₁-C₆-Aminoalkylgruppe stehen; wie 1,3-Diaminopropan, 1,3-Diamino-2-propanol, Spermin oder Spermidin ausgewählt ist;
vorzugsweise die Base aus anorganischen Hydroxiden, wie Natriumhydroxid, Kaliumhydroxid, Lithiumhydroxid, Caesiumhydroxid, Calciumhydroxid, Magnesiumhydroxid, Zinkhydroxid; basischen Aminosäuren, wie Lysin, Arginin, Histidin, Citrullin, Ornithin; Alkanolaminen, wie Monoethanolamin, Diethanolamin, 2-(Dimethylamino)-ethanol, Triethanolamin, Triisopropanolamin, Diisopropanolamin, Monoisopropanolamin; wässrigem Ammoniak; Guanidin carbonat und Mischungen davon ausgewählt ist;
und noch besser die Base aus Arginin, Triethanolamin, Kaliumhydroxid, Natriumhydroxid und Mischungen davon ausgewählt ist.

3. Verwendung nach einem der beiden vorhergehenden Ansprüche, wobei die Spiculosporsäure und/oder die Salze davon in einer kosmetischen Zusammensetzung getragen wird, in der es in einer Menge zwischen 0,5 und 20 Gew.-%, insbesondere zwischen 1 und 15 Gew.-%, spezieller zwischen 1,5 und 10 Gew.-%, bezogen auf das Gesamtgewicht der Zusammensetzung, vorliegt.

4. Verwendung nach Anspruch 3, wobei die kosmetische Zusammensetzung Wasser und/oder ein oder mehrere organische Lösungsmittel, die aus linearen oder verzweigten C₁-C₆-Monoalkoholen, wie Ethanol, Isopropanol, tert-Butanol oder n-Butanol; Polyolen, wie Glycerin, Propylenglykol, Hexylenglykol (oder 2-Methyl-2,4-Pentandiol) und Polyethylenglykolen; Polyolethern, wie Dipropylenglykolmonomethylether; und Mischungen davon ausgewählt sein können, umfasst.

5. Verwendung nach einem der beiden Ansprüche 3 und 4, wobei die kosmetische Zusammensetzung Wasser in einer Menge im Bereich von 30 bis 98 Gew.-%, insbesondere von 40 bis 95 Gew.-%, besser von 50 bis 90 Gew.-%, bezogen auf das Gesamtgewicht der Zusammensetzung, umfasst und/oder die kosmetische Zusammensetzung das organische Lösungsmittel bzw. die organischen Lösungsmittel in einer Menge im Bereich von 0,05 bis 60 Gew.-%, vorzugsweise von 0,5 bis 50 Gew.-% und noch besser von 1 bis 40 Gew.-%, bezogen auf das Gesamtgewicht der kosmetischen Zusammensetzung, umfasst.

6. Verwendung nach einem der Ansprüche 3 bis 5, wobei die kosmetische Zusammensetzung mindestens einen üblichen kosmetischen Inhaltsstoff umfasst, der insbesondere aus pflanzlichen, mineralischen, tierischen oder synthetischen Ölen; flüssigen Fettalkoholen; flüssigen Fettestern; festen Fettsubstanzen und vor allem Wachsen, festen Fettestern, festen Alkoholen; anionischen, kationischen, amphoteren und nichtionischen Tensiden; anionischen, nichtionischen, amphoteren und kationischen Polymeren; Lichtschutzmitteln; Feuchtigkeitsmitteln; Antischuppenmitteln, die von Spiculosporsäure verschieden sind; Antioxidantien; Mitteln zur Bekämpfung von Haarverlust; Perlglanz- und Trübungsmitteln; Weichmachern oder Koaleszenzmitteln; Hydroxysäuren; Füllstoffen; Silikonen und insbesondere Polydimethylsiloxanen (PDMS); Parfümen; Basifizierungsmitteln oder Ansäuerungsmitteln; Aldehyden, DHA; polymeren oder nicht polymeren Verdickern und vor allem assoziativen Polymeren; Konservierungsstoffen; Sequestriermitteln (EDTA und Salzen davon) und Farbmitteln ausgewählt ist.

7. Verwendung nach einem der Ansprüche 3 bis 6, wobei der pH-Wert der kosmetischen Zusammensetzung zwischen 4,5 und 7,5, vor allem zwischen 4,5 und 7 und insbesondere zwischen 4,7 und 6,5 liegt.

8. Verwendung nach einem der Ansprüche 3 bis 7, wobei es sich bei der kosmetischen Zusammensetzung um eine Zusammensetzung für das Haar handelt, die in Form eines Shampoos, einer Creme, eines Aerosol- oder Nichtaerosolschaums, einer Paste, eines Gels, einer Emulsion, einer Lotion oder eines Stifts vorliegen kann.

9. Kosmetisches Behandlungsverfahren zur Beseitigung von Schuppen und/oder zur Verringerung der Schuppenmenge, bei dem man auf das Haar und die Kopfhaut Spiculosporsäure und/oder ein Salz davon als Antischuppenmittel aufbringt.

10. Verfahren nach Anspruch 9, wobei Spiculosporsäure in einer Mischung mit einer organischen oder anorganischen Base verwendet wird, welche vorzugsweise aus
- wässrigem Ammoniak,
- Alkanolaminen, wie Mono-, Di- und Triethanolaminen, Isopropanolamin und 2-Amino-2-methyl-1-propanol,
- oxyethylenierten und/oder oxypropylenierten Ethylendiaminen,
- anorganischen oder organischen Hydroxiden,
- Alkalimetallsilikaten, wie Natriummetasilikaten,
- Aminosäuren, vorzugsweise basischen Aminosäuren, wie Arginin, Lysin, Ornithin, Citrullin und Histidin,
- Carbonaten und Hydrogencarbonaten, insbesondere von einem primären Amin, sekundären Amin oder tertiären Amin, von einem Alkalimetall oder Erdalkalimetall oder von Ammonium, und
- den Verbindungen der folgenden Formel: 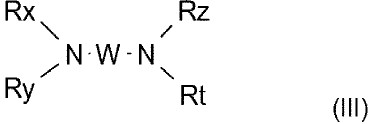 wobei W für einen C₁-C₆-Alkylenrest, der gegebenenfalls durch eine Hydroxylgruppe oder eine C₁-C₆-Alkylgruppe substituiert ist, steht; Rx, Ry, Rz und Rt, die gleich oder verschieden sein können, für ein Wasserstoffatom oder eine C₁-C₆-Alkyl-, C₁-C₆-Hydroxyalkyl- oder C₁-C₆-Aminoalkylgruppe stehen; wie 1,3-Diaminopropan, 1,3-Diamino-2-propanol, Spermin oder Spermidin ausgewählt ist;
vorzugsweise die Base aus anorganischen Hydroxiden, wie Natriumhydroxid, Kaliumhydroxid, Lithiumhydroxid, Caesiumhydroxid, Calciumhydroxid, Magnesiumhydroxid, Zinkhydroxid; basischen Aminosäuren, wie Lysin, Arginin, Histidin, Citrullin, Ornithin; Alkanolaminen, wie Monoethanolamin, Diethanolamin, 2-(Dimethylamino)-ethanol, Triethanolamin, Triisopropanolamin, Diisopropanolamin, Monoisopropanolamin; wässrigem Ammoniak; Guanidin carbonat und Mischungen davon ausgewählt ist;
und noch besser die Base aus Arginin, Triethanolamin, Kaliumhydroxid, Natriumhydroxid und Mischungen davon ausgewählt ist.

11. Verfahren nach einem der beiden Ansprüche 9 und 10, wobei die Spiculosporsäure und/oder die Salze davon in der kosmetischen Zusammensetzung in einer Menge zwischen 0,5 und 20 Gew.-%, insbesondere zwischen 1 und 15 Gew.-%, spezieller zwischen 1,5 und 10 Gew.-%, bezogen auf das Gesamtgewicht der Zusammensetzung, vorliegt.

12. Verfahren nach einem der Ansprüche 9 bis 11, wobei die kosmetische Zusammensetzung Wasser und/oder ein oder mehrere organische Lösungsmittel, die aus linearen oder verzweigten C₁-C₆-Monoalkoholen, wie Ethanol, Isopropanol, tert-Butanol oder n-Butanol; Polyolen, wie Glycerin, Propylenglykol, Hexylenglykol (oder 2-Methyl-2,4-Pentandiol) und Polyethylenglykolen; Polyethern, wie Dipropylenglykolmonomethylether; und Mischungen davon ausgewählt sein können, umfasst;
vor allem wobei die kosmetische Zusammensetzung Wasser in einer Menge im Bereich von 30 bis 98 Gew.-%, insbesondere von 40 bis 95 Gew.-%, besser von 50 bis 90 Gew.-%, bezogen auf das Gesamtgewicht der Zusammensetzung, umfasst und/oder die kosmetische Zusammensetzung das organische Lösungsmittel bzw. die organischen Lösungsmittel in einer Menge im Bereich von 0,05 bis 60 Gew.-%, vorzugsweise von 0,5 bis 50 Gew.-% und noch besser von 1 bis 40 Gew.-%, bezogen auf das Gesamtgewicht der kosmetischen Zusammensetzung, umfasst.

13. Verfahren nach einem der Ansprüche 9 bis 12, wobei die kosmetische Zusammensetzung mindestens einen üblichen kosmetischen Inhaltsstoff umfasst, der insbesondere aus pflanzlichen, mineralischen, tierischen oder synthetischen Ölen; flüssigen Fettalkoholen; flüssigen Fettestern; festen Fettsubstanzen und vor allem Wachsen, festen Fettestern, festen Alkoholen; anionischen, kationischen, amphoteren und nichtionischen Tensiden; anionischen, nichtionischen, amphoteren und kationischen Polymeren; Lichtschutzmitteln; Feuchtigkeitsmitteln; Antischuppenmittel, die von Spiculosporsäure verschieden sind; Antioxidantien; Mitteln zur Bekämpfung von Haarverlust; Perlglanz- und Trübungsmitteln; Weichmachern oder Koaleszenzmitteln; Hydroxysäuren; Füllstoffen; Silikonen und insbesondere Polydimethylsiloxanen (PDMS);
Parfümen; Basifizierungsmitteln oder Ansäuerungsmitteln; Aldehyden, DHA; polymeren oder nicht polymeren Verdickern und vor allem assoziativen Polymeren; Konservierungsstoffen; Sequestriermitteln (EDTA und Salzen davon) und Farbmitteln ausgewählt ist.

14. Verfahren nach einem der Ansprüche 9 bis 13, wobei der pH-Wert der kosmetischen Zusammensetzung zwischen 4 und 7,5, vor allem zwischen 4,5 und 7 und insbesondere zwischen 4,7 und 6,5 liegt.

15. Verfahren nach einem der Ansprüche 9 bis 14, wobei es sich bei der kosmetischen Zusammensetzung um eine Zusammensetzung für das Haar handelt, die in Form eines Shampoos, einer Creme, eines Aerosol- oder Nichtaerosolschaums, einer Paste, eines Gels, einer Emulsion, einer Lotion oder eines Stifts vorliegen kann.

## Revendications

1. Utilisation cosmétique, en particulier pour les cheveux, d'acide spiculisporique et/ou d'un sel de celui-ci en tant qu'agent antipelliculaire.

2. Utilisation selon la revendication 1, dans laquelle l'acide spiculisporique est utilisé en un mélange avec une base organique ou inorganique, de préférence choisie parmi :
- l'ammoniaque,
- les alcanolamines, telles que les mono-, di- et triéthanolamines, l'isopropanolamine et le 2-amino-2-méthylpropan-1-ol,
- les éthylènediamines oxyéthylénées et/ou oxypropylénées,
- les hydroxydes inorganiques ou organiques,
- les silicates de métaux alcalins, tels que les métasilicates de sodium,
- les acides aminés, de préférence les acides aminés basiques, tels que l'arginine, la lysine, l'ornithine, la citrulline et l'histidine,
- les carbonates et les bicarbonates, en particulier d'une amine primaire, amine secondaire ou amine tertiaire, d'un métal alcalin ou métal alcalinoterreux ou d'ammonium et
- les composés de formule suivante : 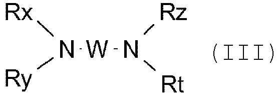 dans lesquels W est un résidu alkylène en C₁-C₆ éventuellement substitué par un groupe hydroxyle ou un groupe alkyle en C₁-C₆ ; Rx, Ry, Rz et Rt, qui peuvent être identiques ou différents, représentent un atome d'hydrogène ou un groupe alkyle en C₁-C₆, hydroxyalkyle en C₁-C₆ ou aminoalkyle en C₁-C₆ ; tels que le 1,3-diaminopropane, le 1,3-diaminopropan-2-ol, la spermine ou la spermidine ;
de préférence, la base est choisie parmi les hydroxydes inorganiques, tels que l'hydroxyde de sodium, l'hydroxyde de potassium, l'hydroxyde de lithium, l'hydroxyde de césium, l'hydroxyde de calcium, l'hydroxyde de magnésium, l'hydroxyde de zinc ; les acides aminés basiques, tels que la lysine, l'arginine, l'histidine, la citrulline, l'ornithine ; les alcanolamines, telles que la monoéthanolamine, la diéthanolamine, le 2-(diméthylamino)éthanol, la triéthanolamine, la triisopropanolamine, la diisopropanolamine, la monoisopropanolamine ; l'ammoniaque ; le carbonate de guanidine ; et les mélanges de ceux-ci ;
et même mieux encore la base est choisie parmi l'arginine, la triéthanolamine, l'hydroxyde de potassium, l'hydroxyde de sodium et les mélanges de ceux-ci.

3. Utilisation selon l'une ou l'autre des revendications précédentes, dans laquelle l'acide spiculisporique et/ou les sels de celui-ci sont véhiculés dans une composition cosmétique dans laquelle ils sont présents en une quantité comprise entre 0,5 et 20 % en poids, en particulier entre 1 et 15 % en poids, plus particulièrement entre 1,5 et 10 % en poids, par rapport au poids total de la composition.

4. Utilisation selon la revendication 3, dans laquelle ladite composition cosmétique comprend de l'eau et/ou un ou plusieurs solvants organiques qui peuvent être choisis parmi les monoalcools en C₁-C₆ linéaires ou ramifiés, tels que l'éthanol, l'isopropanol, le tert-butanol ou le n-butanol ; les polyols, tels que le glycérol, le propylèneglycol, l'hexylèneglycol (ou 2-méthylpentane-2,4-diol) et les polyéthylèneglycols ; les éthers de polyols, tels que l'éther monométhylique de dipropylèneglycol ; et les mélanges de ceux-ci.

5. Utilisation selon l'une ou l'autre des revendications 3 et 4, dans laquelle ladite composition cosmétique comprend de l'eau en une quantité allant de 30 à 98 % en poids, en particulier de 40 à 95 % en poids, mieux de 50 à 90 % en poids, par rapport au poids total de la composition ; et/ou la composition cosmétique comprend le ou les solvants organiques en une quantité allant de 0,05 à 60 %, de préférence de 0,5 à 50 % et mieux encore de 1 à 40 % en poids, par rapport au poids total de la composition cosmétique.

6. Utilisation selon l'une des revendications 3 à 5, dans laquelle ladite composition cosmétique comprend au moins un ingrédient cosmétique usuel, en particulier choisi parmi les huiles végétales, minérales, animales ou synthétiques ; les alcools gras liquides ; les esters gras liquides ; les substances grasses solides et notamment les cires, les esters gras solides, les alcools solides ; les tensioactifs anioniques, cationiques, amphotères et non ioniques ; les polymères anioniques, non ioniques, amphotères et cationiques ; les écrans solaires ; les agents hydratants ; les agents antipelliculaires autres que l'acide spiculisporique ; les antioxydants ; les agents pour la lutte contre la perte des cheveux ; les agents nacrant et opacifiants ; les plastifiants ou les agents de coalescence ; les hydroxyacides ; les charges ; les silicones et en particulier les polydiméthylsiloxanes (les PDMS) ; les parfums ; les agents alcalinisants ou les agents acidifiants ; les aldéhydes, le DHA ; les épaississants polymères ou non polymères et notamment les polymères associatifs ; les conservateurs ; les séquestrants (l'EDTA et les sels de celui-ci) ; les colorants.

7. Utilisation selon l'une des revendications 3 à 6, dans laquelle le pH de ladite composition cosmétique est compris entre 4 et 7,5, notamment entre 4,5 et 7 et en particulier entre 4,7 et 6,5.

8. Utilisation selon l'une des revendications 3 à 7, dans laquelle ladite composition cosmétique est une composition pour les cheveux, qui peut être sous la forme d'un shampooing, d'une crème, d'une mousse aérosol ou non-aérosol, d'une pâte, d'un gel, d'une émulsion, d'une lotion ou d'un stick.

9. Procédé de traitement cosmétique pour l'élimination de pellicules et/ou pour la réduction de la quantité de celles-ci, comprenant l'application, aux cheveux et au cuir chevelu, d'acide spiculisporique et/ou d'un sel de celui-ci en tant qu'agent antipelliculaire.

10. Procédé selon la revendication 9, dans lequel l'acide spiculisporique est utilisé en un mélange avec une base organique ou inorganique, de préférence choisie parmi :
- l'ammoniaque,
- les alcanolamines, telles que les mono-, di- et triéthanolamines, l'isopropanolamine et le 2-amino-2-méthylpropan-1-ol,
- les éthylènediamines oxyéthylénées et/ou oxypropylénées,
- les hydroxydes inorganiques ou organiques,
- les silicates de métaux alcalins, tels que les métasilicates de sodium,
- les acides aminés, de préférence les acides aminés basiques, tels que l'arginine, la lysine, l'ornithine, la citrulline et l'histidine,
- les carbonates et les bicarbonates, en particulier d'une amine primaire, amine secondaire ou amine tertiaire, d'un métal alcalin ou métal alcalinoterreux ou d'ammonium et
- les composés de formule suivante : 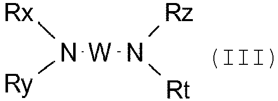 dans lesquels W est un résidu alkylène en C₁-C₆ éventuellement substitué par un groupe hydroxyle ou un groupe alkyle en C₁-C₆ ; Rx, Ry, Rz et Rt, qui peuvent être identiques ou différents, représentent un atome d'hydrogène ou un groupe alkyle en C₁-C₆, hydroxyalkyle en C₁-C₆ ou aminoalkyle en C₁-C₆ ; tels que le 1,3-diaminopropane, le 1,3-diaminopropan-2-ol, la spermine ou la spermidine ;
de préférence, la base est choisie parmi les hydroxydes inorganiques, tels que l'hydroxyde de sodium, l'hydroxyde de potassium, l'hydroxyde de lithium, l'hydroxyde de césium, l'hydroxyde de calcium, l'hydroxyde de magnésium, l'hydroxyde de zinc ; les acides aminés basiques, tels que la lysine, l'arginine, l'histidine, la citrulline, l'ornithine ; les alcanolamines, telles que la monoéthanolamine, la diéthanolamine, le 2-(diméthylamino)éthanol, la triéthanolamine, la triisopropanolamine, la diisopropanolamine, la monoisopropanolamine ; l'ammoniaque ; le carbonate de guanidine ; et les mélanges de ceux-ci ;
et mieux encore la base est choisie parmi l'arginine, la triéthanolamine, l'hydroxyde de potassium, l'hydroxyde de sodium et les mélanges de ceux-ci.

11. Procédé selon l'une ou l'autre des revendications 9 et 10, dans lequel l'acide spiculisporique et/ou les sels de celui-ci sont présents dans la composition cosmétique en une quantité comprise entre 0,5 et 20 % en poids, en particulier entre 1 et 15 % en poids, plus particulièrement entre 1,5 et 10 % en poids, par rapport au poids total de la composition.

12. Procédé selon l'une des revendications 9 à 11, dans lequel ladite composition cosmétique comprend de l'eau et/ou un ou plusieurs solvants organiques qui peuvent être choisis parmi les monoalcools en C₁-C₆ linéaires ou ramifiés, tels que l'éthanol, l'isopropanol, le *tert-*butanol ou le *n*-butanol ; les polyols, tels que le glycérol, le propylèneglycol, l'hexylèneglycol (ou 2-méthylpentane-2,4-diol) et les polyéthylèneglycols ; les éthers de polyols, tels que l'éther monométhylique de dipropylèneglycol ; et les mélanges de ceux-ci ; notamment ladite composition cosmétique comprend de l'eau en une quantité allant de 30 à 98 % en poids, en particulier de 40 à 95 % en poids, mieux encore de 50 à 90 % en poids, par rapport au poids total de la composition ; et/ou la composition cosmétique comprend le ou les solvants organiques en une quantité allant de 0,05 à 60 %, de préférence de 0,5 à 50 % et mieux encore de 1 à 40 % en poids, par rapport au poids total de la composition cosmétique.

13. Procédé selon l'une des revendications 9 à 12, dans lequel ladite composition cosmétique comprend au moins un ingrédient cosmétique usuel, en particulier choisi parmi les huiles végétales, minérales, animales ou synthétiques ; les alcools gras liquides ; les esters gras liquides ; les substances grasses solides et notamment les cires, les esters gras solides, les alcools solides ; les tensioactifs anioniques, cationiques, amphotères et non ioniques ; les polymères anioniques, non ioniques, amphotères et cationiques ; les écrans solaires ; les agents hydratants ; les agents antipelliculaires autres que l'acide spiculisporique ; les antioxydants ; les agents pour la lutte contre la perte des cheveux ; les agents nacrant et opacifiants ; les plastifiants ou les agents de coalescence ; les hydroxyacides ; les charges ; les silicones et en particulier les polydiméthylsiloxanes (les PDMS) ; les parfums ; les agents alcalinisants ou les agents acidifiants ; les aldéhydes, le DHA ; les épaississants polymères ou non polymères et notamment les polymères associatifs ; les conservateurs ; les séquestrants (l'EDTA et les sels de celui-ci) ; les colorants.

14. Procédé selon l'une des revendications 9 à 13, dans lequel le pH de ladite composition cosmétique est compris entre 4 et 7,5, notamment entre 4,5 et 7 et en particulier entre 4,7 et 6,5.

15. Procédé selon l'une des revendications 9 à 14, dans lequel ladite composition cosmétique est une composition pour les cheveux, qui peut être sous la forme d'un shampooing, d'une crème, d'une mousse aérosol ou non-aérosol, d'une pâte, d'un gel, d'une émulsion, d'une lotion ou d'un stick.
